(19)

Europäisches
Patentamt
European
Patent Office
Office européen
des brevets

(11)  **EP 4 451 146 A1**

(12)  # EUROPEAN PATENT APPLICATION

(43) Date of publication:
**23.10.2024 Bulletin 2024/43**

(21) Application number: **23169305.2**

(22) Date of filing: **21.04.2023**

(51) International Patent Classification (IPC):
**G06F 18/22** $^{(2023.01)}$

(52) Cooperative Patent Classification (CPC):
**G06F 18/22**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Gojob**
**13090 Aix-en-Provence (FR)**

(72) Inventors:
• **DELECRAZ, Sébastien**
  **13090 Aix-en-Provence (FR)**

• **ELTARR, Loukman**
  **13090 Aix-en-Provence (FR)**
• **BECUWE, Martin**
  **13090 Aix-en-Provence (FR)**
• **BOUTIN, Nicolas**
  **13090 Aix-en-Provence (FR)**
• **ALLUARD, Oliver**
  **13090 Aix-en-Provence (FR)**

(74) Representative: **Icosa**
**83 avenue Denfert-Rochereau**
**75014 Paris (FR)**

(54)  **COMPUTER-IMPLEMENTED METHOD FOR FAST MATCHING OF ENTITIES FROM DIFFERENT DATASETS**

(57)    The present invention relates to a computer-implemented method and system for matching entities from distinct datasets without directly employing a machine learning model. The method leverages cross-feature computation, features importance computation, and feature trend scores to efficiently and accurately predict matching scores between entities from a first dataset and a second distinct dataset. By reducing energy consumption, memory usage, and computation needs, the inventive method enables faster decision-making, improved responsiveness, and streamlined matching processes across various domains, such as healthcare, finance, human resources management, or e-commerce applications. The inventive method provides a scalable and adaptable solution that can be easily integrated into existing systems or workflows, promoting greater versatility and customization in the matching process and accommodating a wide range of applications and use cases.

FIG. 1

EP 4 451 146 A1

**Description**

**FIELD OF INVENTION**

The present invention relates generally to the field of machine learning and, more specifically, to a computer-implemented method and system for matching entities from distinct datasets.

**BACKGROUND OF INVENTION**

In recent years, machine learning techniques have been increasingly employed to analyze large datasets and identify patterns or relationships between entities within and across these datasets. A common task in various industries is to match entities from different datasets based on their attributes and characteristics. Traditional methods often use machine learning models to compute similarity scores between entities for matching purposes. However, these methods typically require high computational resources, substantial memory usage, and significant energy consumption, making them less suitable for resource-constrained environments or real-time decision-making.

Furthermore, the reliance on machine learning models for computing similarity scores may result in increased latency when applied to large datasets, due to the need to process and analyze vast amounts of data. This may adversely affect the responsiveness and efficiency of systems that require real-time or near-real-time matching results for decision-making purposes, such as in healthcare, finance, human resources management, or e-commerce applications.

There is a need for a method that can efficiently predict matching scores without directly using the machine learning model, thereby reducing energy consumption, memory usage, and computation needs while providing an estimate for the matching result that is accurate enough to give prediction trends and support decision-making processes.

**SUMMARY**

This invention thus relates to a computer-implemented method for matching entities from a first dataset and a second distinct dataset, the method comprising: a) receiving a first training dataset corresponding to information of first entities, wherein the first entity information includes various characteristics relating to the entities and their attributes; b) receiving a second training dataset corresponding to information of second entities, wherein the second entity information includes various characteristics relating to the entities and their attributes;
c) computing cross-features between the entities of the first dataset and the entities of the second dataset using a cross-feature computation (CFC) process; d) training a machine learning (ML) model to compute a similarity score between an entity of the first dataset and an entity of the second dataset, based on the cross-features calculated in the CFC process and annotations; e) determining features importance of the trained ML model using a features importance computation (FIC) process, wherein the process calculates the Shapley values of the features, and the curve of the Accumulated Local Effects (ALE) for the features; g) receiving a new example for which a match is to be determined, said new example being related to a first entity or a second entity, and computing cross-features using the CFC process for said new example; h) for each cross-feature computed, selecting examples from the training dataset similar to the new example and computing a feature trend score based on the Shapley values and ALE values of the selected examples; i) computing and outputting a matching result for the new example based on the computed feature trend scores.

The "matching result" outputted by the computer-implemented method according to the invention is thus an estimate of the prediction of the machine learning model. In other words, the matching result is an estimate of the similarity score that the machine learning model would have outputted for the same entities.

The present invention provides a computer-implemented method for matching entities from a first dataset and a second distinct dataset without directly employing a machine learning model. By leveraging cross-feature computation, features importance computation, and feature trend scores, the method offers an efficient and accurate prediction of matching scores, enabling faster decision-making and reducing energy consumption, memory usage, and computation needs.

The inventive method can be applied across various domains, where it may offer several advantages over traditional matching techniques. For example, the method can improve the responsiveness of systems requiring real-time or near-real-time matching results, streamline the matching process, and facilitate the identification of relevant matches or relationships between entities in large datasets more quickly and effectively than the prior art.

In addition, the inventive method provides a scalable and adaptable solution that can be easily integrated into existing systems or workflows, promoting greater versatility and customization in the matching process and accommodating a wide range of applications and use cases.

In an embodiment, the matching result is a normalized weighted average of the ALE values of the cross-features weighted by the mean of the absolute Shapley values of the selected examples.

In an embodiment, the examples of the training dataset are selected by taking a predetermined number of examples having the closest value for the considered cross-feature of the new example.

In an embodiment, the predetermined number is less than or equal to 10% of the number of distinct pairs of entities in the first and the second dataset.

In an embodiment, the cross-feature computation process further comprises calculating distances, similarities, or correlations between characteristics of first entities in the first dataset and characteristics of second entities in the second dataset.

In an embodiment, the machine learning model is selected from the group consisting of support vector machines, decision trees, random forests, neural networks, k-nearest neighbors, and gradient boosting machines. Other type of machine learning models may be used in the context of the present invention.

In an embodiment, step i) further comprises generating a confidence index for the matching result.

In an embodiment, the first dataset corresponds to patient health information, including various characteristics related to the patient's health condition, such as for example symptoms, demographic information, medical history, laboratory test results, and imaging data, and the second dataset corresponds to known disease cases, including various characteristics related to the diseases, thereby applying the method to identify potential diseases diagnoses based on the highest similarity scores between patient health data and know diseases cases to assist professionals in making a diagnosis or recommend further tests.

In an embodiment, the first dataset corresponds to worker information, including various characteristics relating to the worker's skills, availability, and other relevant attributes, and the second dataset corresponds to available job offer information, including various characteristics relating to the job offer, such as for example required skills, location, and other relevant attributes, thereby applying the method to match workers with job offers based on the computed matching results.

An other object of the invention relates to a system for matching entities from a first dataset and a second distinct dataset, the system comprising: a) a data input module configured to receive a first training dataset corresponding to information of first entities and a second training dataset corresponding to information of second entities, wherein the first entity information and the second entity information include various characteristics relating to the entities and their attributes; b) a cross-feature computation (CFC) module configured to compute cross-features between the entities of the first dataset and the entities of the second dataset; c) a machine learning (ML) module configured to train an ML model to compute a similarity score between an entity of the first dataset and an entity of the second dataset, based on the cross-features calculated by the CFC module and annotations; d) a features importance computation (FIC) module configured to determine features importance of the trained ML model by calculating the Shapley values of the features and the curve of the Accumulated Local Effects (ALE) for the features; e) a matching module configured to receive a new example for which a match is to be determined, compute cross-features using the CFC module for the new example, and compute and output a matching result for the new example based on the computed feature trend scores.

The invention also relates to a non-transitory computer-readable medium storing computer program instructions, which when executed, cause a processor to perform a computer-implemented method as described before.

## DEFINITIONS

As used in the context of the present invention, the term **"machine learning model"** refers to a computational algorithm or a set of algorithms that are capable of learning patterns or relationships in input data through a training process. The learned patterns or relationships can then be used to make predictions, classifications, or recommendations for new, previously unseen data. Machine learning models can be based on various techniques, including but not limited to supervised learning, unsupervised learning, semi-supervised learning, reinforcement learning, and deep learning. Examples of machine learning models include, but are not limited to, decision trees, support vector machines, artificial neural networks, k-means clustering, principal component analysis, and reinforcement learning agents. In the context of this invention, a machine learning model is used to compute similarity scores between entities based on their attributes and characteristics, as derived from cross-features computed through a cross-feature computation process.

As used in the context of the present invention, the term **"cross-feature"** refers to a derived feature or attribute that represents a combination, interaction, or relationship between one or more features from different entities in distinct datasets. Cross-features are generated through a cross-feature computation (CFC) process, which may involve various mathematical or computational operations, such as for example addition, subtraction, multiplication, division, or other transformations, depending on the nature of the original features and the desired outcome of the matching process.

Cross-features serve as a means to compare and analyze entities from separate datasets by capturing and quantifying the similarities, dissimilarities, or relationships between their attributes or characteristics. In the context of this patent application, cross-features are employed to train a machine learning model to compute similarity scores between entities from a first dataset and a second distinct dataset. These similarity scores are subsequently used to determine the matching results for new examples based on the computed feature trend scores, without directly employing the machine learning model.

As used in the context of the present invention, the **"Shapley value of a feature"** is computed by considering all possible permutations of features and measuring the marginal contribution of that feature to the prediction outcome when added to a subset of features. The average of these marginal contributions across all permutations provides an unbiased and mathematically fair estimate of the feature's importance, taking into account the interaction effects between features. In this patent application, Shapley values are used in the features importance computation process to assess and quantify the relative importance of different features in determining the similarity scores between entities in the first and second datasets.

As used in the context of the present invention, the term **"accumulated local effects (ALE) value of a feature"** refers to a measure of the effect or contribution of a specific feature on the prediction outcome of a machine learning model, while accounting for the interactions between that feature and other features in the model. The ALE value is computed by estimating the average change in the model's prediction as the value of the feature of interest varies while keeping the values of other features fixed. This is achieved by calculating the partial dependence of the model's prediction on the feature of interest while integrating over the distribution of other features in the dataset.

In contrast to other feature importance methods, such as partial dependence plots, ALE values offer a more robust and accurate estimation of a feature's effect in the presence of correlated features. By considering local changes in the prediction outcome, the ALE value provides insights into the relationship between the feature of interest and the model's prediction across different regions of the feature space.

In the present invention, accumulated local effects values of features are used in the features importance computation process to assess and quantify the local impact of different features in determining the similarity scores between entities in the first and second datasets. This information, in combination with the Shapley values, aids in computing a feature trend score for each cross-feature to estimate matching results without directly using the machine learning model.

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

## BRIEF DESCRIPTION OF THE DRAWINGS

FIG. 1 is a flowchart illustrating the steps of the computer-implemented method for matching entities from distinct datasets according to one embodiment of the present invention.

FIG. 2 is a block diagram illustrating the system for matching entities from distinct datasets according to one embodiment of the present invention.

## DETAILED DESCRIPTION

In the following detailed description, reference is made to the accompanying drawings, which form a part of the description, and in which are shown by way of illustration, specific embodiments of the invention. These embodiments are described in sufficient detail to enable those skilled in the art to practice the invention, and it is to be understood that other embodiments may be utilized, and that structural, logical, and electrical changes may be made without departing from the spirit and scope of the present invention.

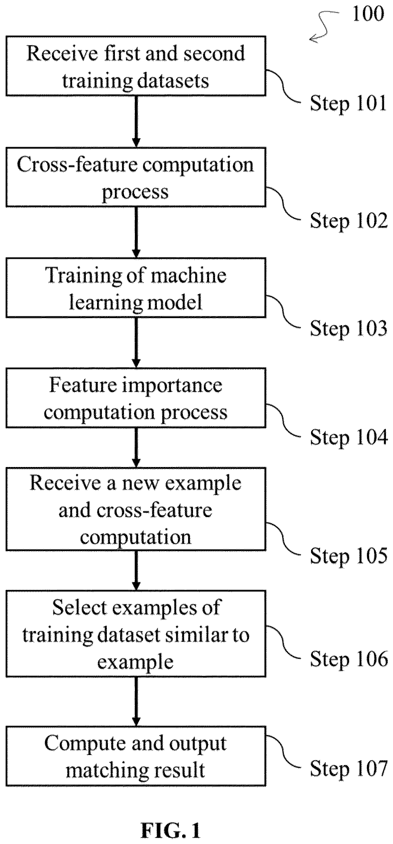

FIG. 1 is a flowchart illustrating the steps of the computer-implemented method 100 for matching entities from distinct datasets according to one embodiment of the present invention.

The method begins at step 101, where a first training dataset and a second training dataset are received. These datasets include information about first entities and second entities, respectively.

The datasets or any of the data processed in the present invention can take the form of storage resources available from any kind of appropriate storage means, which can be notably a RAM or an EEPROM (Electrically-Erasable Programmable Read-Only Memory) such as a Flash memory, possibly within an SSD (Solid-State Disk). The datasets can be received from a communication network.

At step 102, the cross-feature computation (CFC) process is performed to compute cross-features between the entities of the first dataset and the entities of the second dataset. This process captures and quantifies the similarities, dissimilarities, or relationships between the attributes or characteristics of the entities in the distinct datasets. It may output a plurality of vectors for each entity of the first and second dataset, for example a vector for each combination of entities in the first and the second dataset.

At step 103, a machine learning (ML) model is trained to compute similarity scores between the entities of the first dataset and the entities of the second dataset, based on the cross-features calculated in the CFC process and annotations. Annotations are similarity scores manually provided to train the machine learning model. The machine learning model may be selected from the group consisting of support vector machines, decision trees, random forests, neural networks, k-nearest neighbors, and gradient boosting machines.

At step 104, the features importance computation (FIC) process is performed to determine the features importance of the trained ML model. The process calculates the Shapley values of the features and the curve of the Accumulated Local Effects (ALE) for the features. These values are used to assess and quantify the relative importance of different features in determining the similarity scores between the entities in the first and second datasets.

It has to be noted that steps 101, 102, 103 and 104 may be performed only once, thus limiting the computation time needed. Indeed, the results of the features importance computation process (Shapley values and ALE values) at step 104 can be stored in a memory (e.g. in a matrix format) for subsequent use at steps 106 and 107.

At step 105, a new example is received for which a match is to be determined. The new example can be related to a first entity or a second entity. Cross-features are computed for the new example using the CFC process. In particular, cross-features may be computed between the new example as being a first entity and all entities of the second dataset, to provide, in the end, matching results for the new example with respect to all entities of the second dataset. The same reasoning is also possible for a new example as being a second entity.

At step 106, for each cross-feature computed for the new example, examples from the training dataset similar to the new example are selected. If the example is a first entity, the examples are selected in the first dataset, while if the example is a second entity, the examples are selected in the second dataset. A feature trend score is computed based on the Shapley values and ALE values of the selected examples.

The selection of examples from the training dataset can be based on taking a predetermined number of examples having the closest value for the considered cross-feature of the new example. Any rule can be used to select the examples for the training dataset similar to the new example. For example, by taking a predetermined number of examples having the closest value for the considered cross-feature of the new example (e.g. the predetermined number may be less than or equal to 10% of the number of distinct pairs of entities in the first and the second dataset). One can also use a specific distribution rule.

At step 107, a matching result M is computed and outputted for the new example based on the computed feature trend scores. It should be understood that several matching results M may be computed and outputted between the new example and several or all entities of the other dataset, for example to select, in a subsequent step, the entity of the other dataset having the highest matching result.

The matching result M can be a normalized weighted average of the ALE values of the cross-features weighted by the mean of the absolute Shapley values of the selected examples. A confidence score may also be outputted together with the matching result.

In an example, the matching result M may be given by the following formula:

$$M = \frac{1}{n * \sum_{i=1}^{n} SHAP(i)} \sum_{i=1}^{n} SHAP(i) \times ALE(x_i)$$

Where n is the number of features computed by the CFC process, i is the index of the feature considered, $x_i$ is the value of feature i of the new example, $ALE(x_i)$ is the ALE value for $x_i$, and $SHAP(i)$ is defined by the formula below:

$$SHAP(i) = \frac{1}{m} \sum_{j=1}^{m} \|S(i,j)\|$$

Where m is the number of examples selected in the training dataset, and $S(i,j)$ is the Shapley Value of feature i for example j in the training dataset computed using the FIC process.

In an embodiment, $SHAP(i) \times ALE(x_i)$ corresponds to a feature trend score.

It has to be noted that steps 105, 106 and 107 may thus be repeated for several new examples without repeating steps 101, 102, 103 and 104.

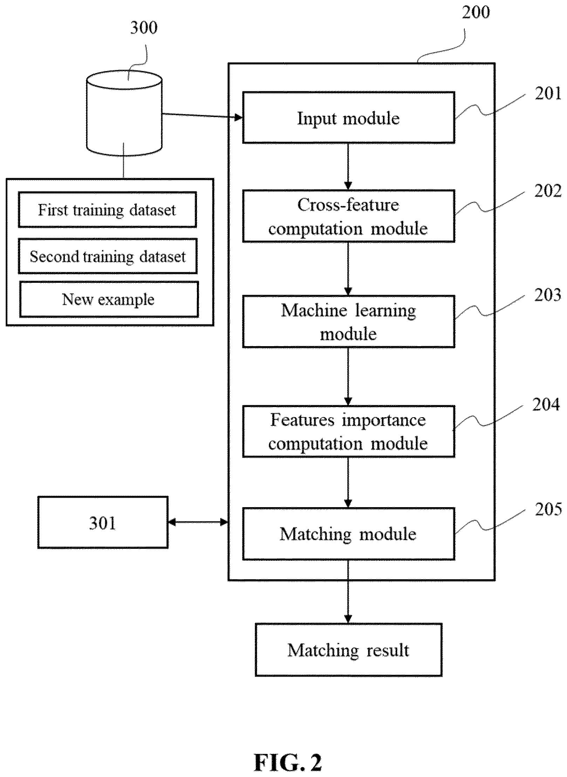

FIG. 2 is a block diagram illustrating the system 200 for matching entities from distinct datasets according to one embodiment of the present invention. The system 200 comprises a data input module 201, a cross-feature computation (CFC) module 202, a machine learning (ML) module 203, a features importance computation (FIC) module 204, and a matching module 205.

The data input module 201 is configured to receive a first training dataset corresponding to information of first entities and a second training dataset corresponding to information of second entities, wherein the first entity information and the second entity information include various characteristics relating to the entities and their attributes. The data input module 201 can receive data from local or remote database(s) 300.

The cross-feature computation (CFC) module 202 is configured to compute cross-features between the entities of the first dataset and the entities of the second dataset.

The machine learning (ML) module 203 is configured to train an ML model to compute a similarity score between an entity of the first dataset and an entity of the second dataset, based on the cross-features calculated by the CFC module and annotations.

The features importance computation (FIC) module 204 is configured to determine the features importance of the trained ML model by calculating the Shapley values of the features and the curve of the Accumulated Local Effects (ALE) for the features.

The matching module 205 is configured to receive a new example for which a match is to be determined, compute cross-features using the CFC module for the new example, and compute and output a matching result for the new example based on the computed feature trend scores.

In an embodiment, the system 200 is interacting with a user interface 301, via which information can be entered and retrieved by the user. The user interface 301 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

The system and method described herein can be implemented in various industries and applications, including healthcare, finance, human resources management, and e-commerce, to name a few. By leveraging cross-feature computation, features importance computation, and feature trend scores, the present invention provides an efficient and accurate prediction of matching scores without directly employing a machine learning model, enabling faster decision-making and reducing energy consumption, memory usage, and computation needs.

**Examples**

A first example of implementation of the invention is in healthcare industry. According to the first example, the first dataset corresponds to patient health information, including various characteristics related to the patient's health condition, such as for example symptoms, demographic information, medical history, laboratory test results, and imaging data, and the second dataset corresponds to known disease cases, including various characteristics related to the diseases, thereby applying the method to identify potential diseases diagnoses based on the highest matching scores between patient health data and know diseases cases to assist professionals in making a diagnosis or recommend further tests.

This implementation permits to obtain a quick screening of several diseases based on information provided related to the patient, when a patient presents to a healthcare provider. Thus, the invention permits quick orientation of the patient depending of the diseases ranking, for example useful in emergency care units.

A second example of implementation of the invention is in the human resources management industry. According to the second example, the first dataset corresponds to worker information, including various characteristics relating to the worker's skills, availability, and other relevant attributes, and the second dataset corresponds to available job offer information, including various characteristics relating to the job offer, such as for example required skills, location, and other relevant attributes, thereby applying the method to match workers with job offers.

This implementation permits to obtain a quick ranking of job offers for a workers seeking a job.

**Technical Effects and Advantages**

The present invention offers several technical effects and advantages over traditional entity matching methods. It can be also used as a complement of traditional entity matching methods to provide a quick estimate of a matching result.

Some of these advantages include:

Improved efficiency: By computing feature trend scores and avoiding the direct use of the machine learning model, the present invention reduces the computational resources, memory usage, and energy consumption required for matching entities from distinct datasets.

Faster decision-making: The inventive method enables real-time or near-real-time matching results, which can be particularly useful for applications that require quick and responsive decision-making processes, such as in healthcare, human resources management, finance, or e-commerce.

Scalability: The present invention provides a scalable and adaptable solution that can be easily integrated into existing systems or workflows, allowing for greater customization and accommodating a wide range of applications and use cases.

Enhanced accuracy: The method leverages features importance computation, including Shapley values and Accumulated Local Effects (ALE) values, to assess and quantify the relative importance and local impact of different features in determining the similarity scores between entities in the first and second datasets. This information aids in computing a feature trend score for each cross-feature, enabling accurate matching results without directly using the machine learning model.

Versatility: The inventive method can be applied across various domains and industries, making it highly versatile and valuable for different applications, such as healthcare, finance, human resources management, and e-commerce, among others.

**Claims**

1. A computer-implemented method (100) for matching entities from a first dataset and a second distinct dataset, the method comprising:

    a) receiving a first training dataset (101) corresponding to information of first entities, wherein the first entity information includes various characteristics relating to the first entities and their attributes;
    b) receiving a second training dataset (101) corresponding to information of second entities, wherein the second entity information includes various characteristics relating to the second entities and their attributes;
    c) computing cross-features (102) between the entities of the first dataset and the entities of the second dataset using a cross-feature computation (CFC) process;
    d) training a machine learning (106) model to compute a similarity score between an entity of the first dataset and an entity of the second dataset, based on the cross-features calculated in the CFC process and annotations;
    e) determining features importance (104) of the trained ML model using a features importance computation (FIC) process, wherein the process calculates the Shapley values of the features, and the curve of the Accumulated Local Effects (ALE) for the features;
    g) receiving a new example (105) for which a match is to be determined, said new example being related to a first entity or a second entity, and computing cross-features using the CFC process for said new example;
    h) for each cross-feature computed, selecting examples (106) from the training dataset similar to the new example and computing a feature trend score based on the Shapley values and ALE values of the selected examples;
    i) computing and outputting a matching result (107) for the new example based on the computed feature trend scores.

2. The computer-implemented method of claim 1, wherein the matching result is a normalized weighted average of the ALE values of the cross-features weighted by the mean of the absolute Shapley values of the selected examples.

3. The computer-implemented method of any of claims 1 or 2, wherein the examples of the training dataset are selected by taking a predetermined number of examples having the closest value for the considered cross-feature of the new example.

4. The computer-implemented method of any of claims 1 to 3, wherein the predetermined number is less than or equal to 10% of the number of distinct pairs of entities in the first and the second dataset.

5. The computer-implemented method of any of claims 1 to 4, wherein the cross-feature computation process (104) further comprises calculating distances, similarities, or correlations between characteristics of first entities in the first dataset and characteristics of second entities in the second dataset.

6. The computer-implemented method of any of claims 1 to 5, wherein the machine learning model is selected from the group consisting of support vector machines, decision trees, random forests, neural networks, k-nearest neighbors, and gradient boosting machines.

7. The computer-implemented method of any of claims 1 to 6, wherein step i) further comprises generating a confidence index for the matching result.

8. The computer-implemented method of any of claims 1 to 7, wherein the first dataset corresponds to patient health information, including various characteristics related to the patient's health condition, such as for example symptoms, demographic information, medical history, laboratory test results, and imaging data, and the second dataset corresponds to known disease cases, including various characteristics related to the diseases, thereby applying the method to identify potential diseases diagnoses based on the highest matching results between patient health data and know diseases cases to assist professionals in making a diagnosis or recommend further tests.

9. The computer-implemented method of any of claims 1 to 8, wherein the first dataset corresponds to worker information, including various characteristics relating to the worker's skills, availability, and other relevant attributes, and the second dataset corresponds to available job offer information, including various characteristics relating to the job offer, such as for example required skills, location, and other relevant attributes, thereby applying the method to match workers with job offers based on the computed matching results.

10. A system (200) for matching entities from a first dataset and a second distinct dataset, the system comprising:

   a) a data input module (201) configured to receive a first training dataset corresponding to information of first entities and a second training dataset corresponding to information of second entities, wherein the first entity information and the second entity information include various characteristics relating to the entities and their attributes;
   b) a cross-feature computation module (202) configured to compute cross-features between the entities of the first dataset and the entities of the second dataset;
   c) a machine learning module (203) configured to train an ML model to compute a similarity score between an entity of the first dataset and an entity of the second dataset, based on the cross-features calculated by the CFC module and annotations;
   d) a features importance computation (FIC) module (204) configured to determine features importance of the trained ML model by calculating the Shapley values of the features and the curve of the Accumulated Local Effects (ALE) for the features;
   e) a matching module (205) configured to receive a new example for which a match is to be determined, compute cross-features using the CFC module for the new example, and compute and output a matching result for the new example based on the computed feature trend scores.

11. A non-transitory computer-readable medium storing computer program instructions, which when executed, cause a processor to perform a computer-implemented method (100) according to any of claims 1 to 9.

**FIG. 1**

FIG. 2

Europäisches
Patentamt

European
Patent Office

Office européen
des brevets

# EUROPEAN SEARCH REPORT

Application Number

EP 23 16 9305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GU YIFAN ET AL: "Structure-aware siamese graph neural networks for encounter-level patient similarity learning", JOURNAL OF BIOMEDICAL INFORMATICS, ACADEMIC PRESS, NEW YORK, NY, US, vol. 127, 16 February 2022 (2022-02-16), XP086979408, ISSN: 1532-0464, DOI: 10.1016/J.JBI.2022.104027 [retrieved on 2022-02-16] * abstract * * figures 1,2 * ----- | 1-11 | INV. G06F18/22 |
| X | GAO JUNYI BUAAGJY@BUAA EDU CN ET AL: "COMPOSE: Cross-Modal Pseudo-Siamese Network for Patient Trial Matching", PROCEEDINGS OF THE 26TH ACM SIGKDD INTERNATIONAL CONFERENCE ON KNOWLEDGE DISCOVERY & DATA MINING, ACMPUB27, NEW YORK, NY, USA, 23 August 2020 (2020-08-23), pages 803-812, XP058461008, DOI: 10.1145/3394486.3403123 ISBN: 978-1-4503-7998-4 * abstract * * figure 1 * ----- | 1-11 | TECHNICAL FIELDS SEARCHED (IPC) G06F |

The present search report has been drawn up for all claims

| Place of search | Date of completion of the search | Examiner |
|---|---|---|
| The Hague | 2 October 2023 | Meier, Ueli |

EPO FORM 1503 03.82 (P04C01)